# EUROPEAN PATENT SPECIFICATION

(11) **EP 2 985 233 B1**
(45) Date of publication and mention of the grant of the patent: **22.03.2017**
(21) Application number: 15002404.0
(22) Date of filing: 12.08.2015
(51) Int. Cl.: B65B 31/04, B65B 57/10, G01N 27/12

(54) **FOOD STORAGE APPLIANCE WITH MOISTURE SENSOR**
NAHRUNGSMITTELLAGERUNGSVORRICHTUNG MIT FEUCHTIGKEITSSENSOR
APPAREIL DE STOCKAGE D'ALIMENTS AVEC CAPTEUR D'HUMIDITÉ

(30) Priority: 12.08.2014 US 201462036384 P
(43) Date of publication of application: 17.02.2016
(73) Proprietor: Sunbeam Products, Inc., Boca Raton, FL 33431 (US)
(72) Inventor: Owens, David, Boynton Beach, Florida 33472 (US)
(74) Representative: Vossius, Tilman

(56) References cited:
- US-A1- 2005 022 474
- US-A1- 2005 166 671

## Description

### CROSS-REFERENCE TO RELATED APPLICATION

The present application claims the benefit of U.S. Provisional Patent Application No. 62/036,384 filed August 12, 2014, entitled "Food Storage Appliance Moisture Sensor Solution".

### FIELD OF THE INVENTION

The invention relates to food preservation, and more particularly to an improved food storage appliance having a sensor to detect liquid in a food storage container before the liquid is drawn into the vacuum trough.

### BACKGROUND OF THE INVENTION

Vacuum packaging appliances that evacuate air from containers holding food are becoming increasingly popular with households for food preservation and storage. The removal of the air delays spoilage and extends the life of the food. The appliances are typically used in conjunction with bag material that constitutes the container holding the food. After the food is inserted in the storage bag, the storage bag is fully sealed by applying heat and pressure to the remaining cut edges. A vacuum may be applied to evacuate air from the storage bag before it is fully sealed. Liquid in the storage bag is typically drawn into the appliance and may be directed into a cavity or a tray for discarding later. US 2005/022474 A1 discloses a known vacuum packaging appliance.

### SUMMARY OF THE INVENTION

In an embodiment, there is provided a food storage appliance including a housing, an elongated vacuum trough disposed in the housing, one or more electronic components including a vacuum motor fluidly connected to the vacuum trough and a heat sealing element disposed in proximity of the vacuum trough, an sensor disposed on the housing in proximity to the vacuum trough configured to generate an electrical signal if liquid is detected in a food storage container, the food storage container having a portion inserted into the vacuum trough prior to an evacuation operation, the sensor detecting liquid before it is drawn into the vacuum trough during the evacuation operation, and an integrated electronic circuit connected to the sensor configured to receive the electrical signal and configured to control one of the vacuum motor or the heat sealing element when liquid is detected in the food storage container.

In an embodiment, there is provided a sensor for a food preservation appliance for detecting liquid in a food preservation container being processed by the food preservation appliance including a substrate comprised of a non-conducting material, a first trace comprised of a conducting material etched onto to the substrate having a plurality of first appendages extending perpendicularly therefrom and spaced along a lineal length of the first trace, a second trace comprised of a conducting material etched onto the substrate separated from the first trace by a first space, the second trace having a plurality of second appendages extending perpendicularly therefrom and spaced along a lineal length of the second trace, the second plurality of appendages interleaved with and separated by a second space from the first plurality of appendages, and a logic circuit electrically connected to the first and second traces, the logic circuit providing a first signal to the first trace and receiving a second signal from the second trace when liquid being evacuated from within the food preservation container by the food preservation appliance is disposed in proximity to the first and second spaces.

In an embodiment, there is provided a method of storing and preserving food in a food storage container using a food storage including the steps of: inserting a portion of the food storage container into a vacuum trough of the food storage machine, commencing evacuating the food storage container using a vacuum motor disposed in the food storage machine fluidly connected to the vacuum trough, using a sensor to generate a signal if liquid is detected in the food storage container prior to the liquid being drawn into the vacuum trough, using a logic circuit to compare the generated signal to a threshold signal, and using the logic circuit to control one or both of the vacuum motor evacuating the food container or a heat sealing element disposed in proximity of the vacuum trough to heat seal the food storage container.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is a perspective view of an embodiment of a food storage appliance;
FIG. 2 is an elevated front perspective view of the food storage appliance of FIG. 1;
FIG. 3 is a block diagram of the major electronic components of the food storage appliance of FIG. 1;
FIG. 4 is an illustration showing the intended use of the food storage appliance of FIG. 1;
FIG. 5 is an illustration of a sensor for detecting liquids for use with the food storage appliance of FIG. 1; and
FIG. 6 is an illustration of the operation of the sensor of FIG. 5.

### DETAILED DESCRIPTION OF THE INVENTION

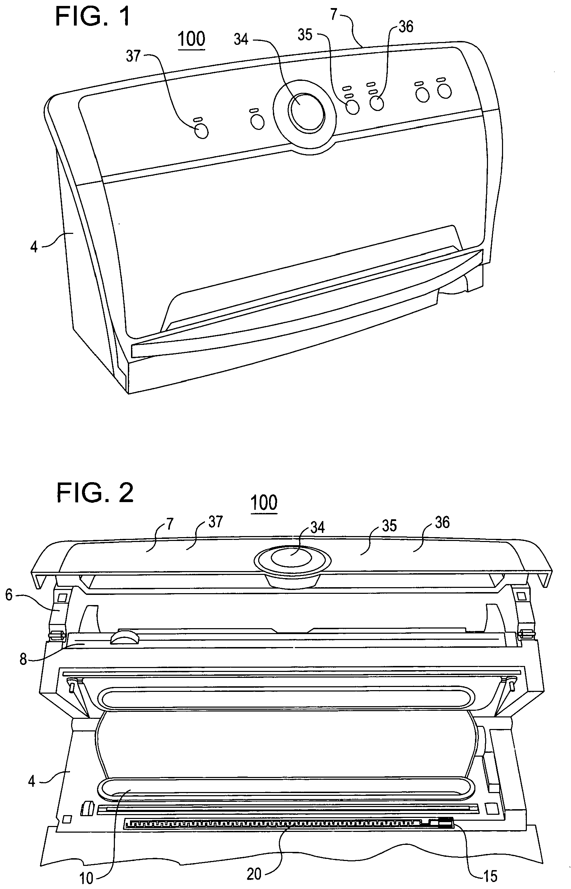

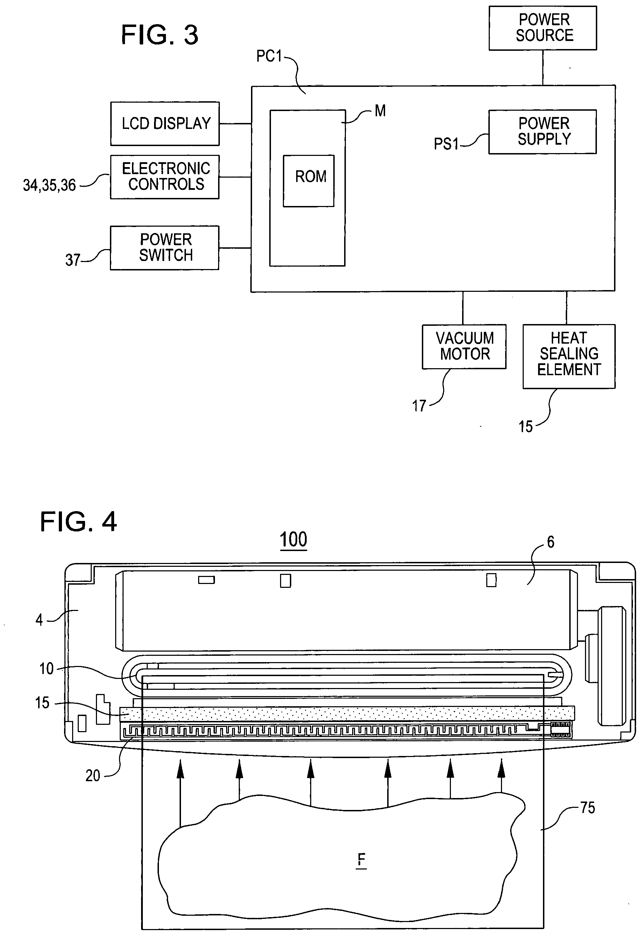

Referring now to FIGS. 1-4 of the drawings, there is illustrated an embodiment of a food storage appliance 100 which includes a housing 4, a packaging film compartment 6, a lid 7, a cutting mechanism 8, an elongated vacuum trough 10, an elongated heat sealing element 15 and a vacuum motor 17. The housing 4 encloses the packaging film compartment 6 covered by the lid 7 and the cutting mechanism 8 (inside the compartment 6) for cutting a length of packaging film stored on a roll in the packaging film compartment 6. The housing 4 includes a vacuum compartment including an elongated vacuum trough 10 for receiving an unsealed end of a food packaging container 75 (see FIG. 4) formed from a length of packaging film cut from the roll of packaging film. The heat sealing element 15 may be disposed on the housing 4 in front of the vacuum trough 10. An elongated sensor 20 is disposed in front of the heat sealing element 15 for detecting moisture or liquid in the food packaging container 75 when one end us inserted into the vacuum trough 10 during an evacuation and sealing operation. The sensor 20 generates an electrical signal when moisture or liquid is detected in the food packaging container 75 for controlling the operation of the food storage appliance 100 as described hereinbelow.

In an embodiment, the food preservation containers 75 may be pre-formed and sealed on three edges at the factory. Alternately, food packaging containers 75 may be pre-sealed along two edges at the factory and formed into a roll and stored in the packaging film compartment 6 as described for dispensing a length at a time. A third edge may be sealed by the user after being cut from the roll by inserting into the vacuum trough 10 and energizing the heat sealing element 15. In either scenario, a fully formed food packaging container 75 containing a food item F may be evacuated and sealed on the remaining unsealed end by inserting it into the vacuum trough 10 and energizing the vacuum motor 17 and/or the heat sealing element 15 via electronic controls on the housing 4 as described hereinbelow.

In particular, the electronic controls includes a user interface for controlling various functions of the food storage appliance 100. The electronic controls may include exteriorly exposed buttons (switches) 34, 35, 36, and 37 for access by the user. For example, the button 34 may operate both the vacuum motor 17 and the heat sealing element 15. Alternately, the button 35 may operate the heat sealing element 15 only. Alternately, the button 36 may operate the vacuum motor 17 only. Finally, the button 37 may control electrical power being provided to the electronic controls and the electronic components within housing 4 as described below.

Within the housing 4, the electronic components may include a microprocessor M mounted on a printed circuit board PC 1 with an operating control program stored in ROM that controls the vacuum motor 17 and the heat sealing element 15, as discussed herein. The electronic components may also include other conventional components such as a power circuit PS 1, an input interface circuit (not shown), an output interface circuit (not shown), and one or more storage devices ME, such as a ROM (Read Only Memory) device and a RAM (Random Access Memory) device. The power circuit PS1 is connected to an AC or DC power source and directs power to the motors, switches, sensors, etc. described herein, as well as provide power to other circuits and components of the electronic controls. The input interface circuit can be electrically connected to the buttons 34, 35, 36 and 37 for user control. The output interface circuit can be electrically connected to a LCD screen. The storage device ME stores processing results and control programs that are run by the microprocessor circuit M. The electronic controls are capable of selectively controlling any of the vacuum motor 17 and the heat sealing element 10 in accordance with the control program. It will be apparent to those skilled in the art from this disclosure that the precise structure and algorithms for the control panel can be any combination of hardware and software that will carry out the functions of the present invention.

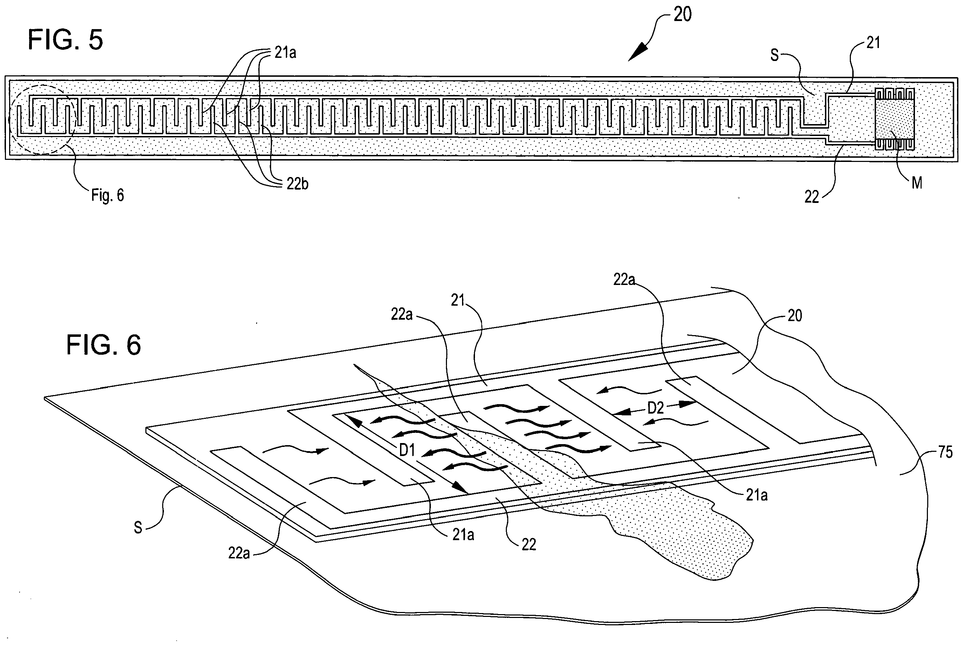

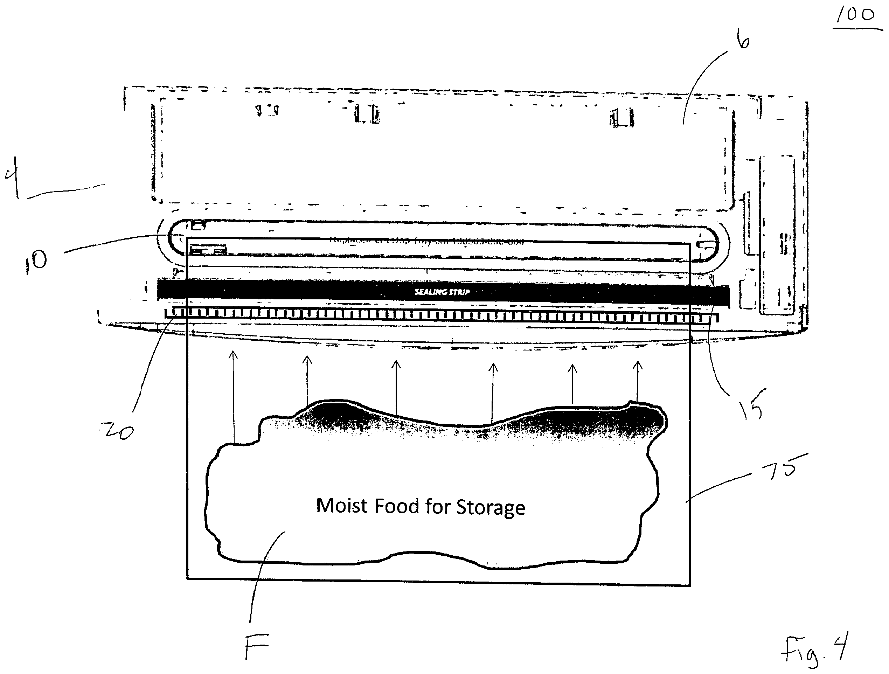

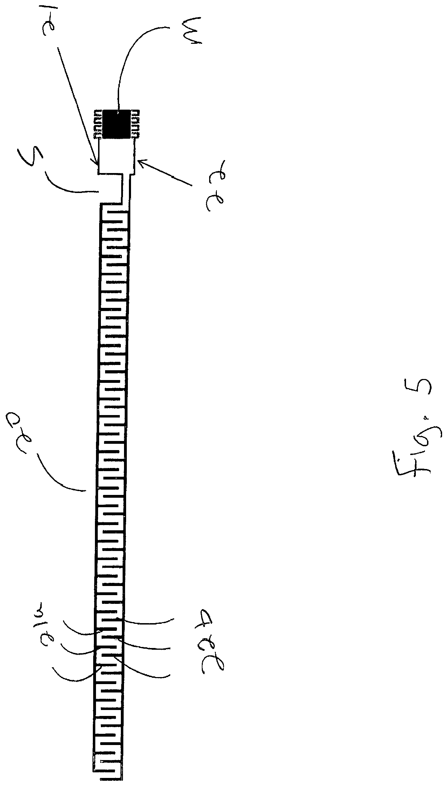

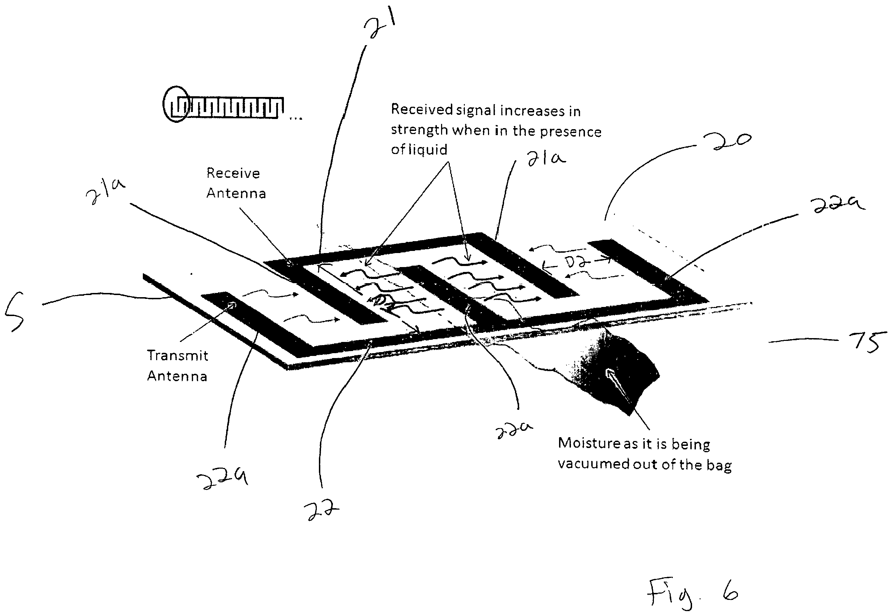

Referring now to FIGS. 5 and 6, there is illustrated an embodiment of a liquid or moisture sensor 20 that is electrically connected to a logic circuit or an integrated circuit such as the microprocessor M described above. The sensor 20 includes two elongated electrically conductive traces 21, 22 disposed adjacent one another etched onto a non-conductive substrate S. The traces 21, 22 may be comprised of a conductive material such as metal including but not limited to copper, gold, silver and aluminum. The traces 21, 22 are spaced apart from one another a small distance D1 forming a dielectric barrier therebetween. One of the traces 21 is a transmitting antenna fed a signal by the microprocessor M. The other trace 22 is a receiving antenna that receives electromagnetic signals generated by trace 21 and feeds the received signal back to the microprocessor M. Each of the traces 21, 22 have discrete appendages 21a, 22a extending perpendicularly therefrom along their length at equally spaced intervals such that the appendages 21a, 22a interleave with one another but spaced at a small distance D2 apart forming a dielectric barrier.

In an embodiment, normally when electrical power to the food storage machine 100 is turned on at button 37, the microprocessor M generates no signal or a weak signal provided to trace 21 and appendages 21a. As such, no signal or a weak signal is transmitted by the trace 21 and appendages 21a and no signal or a very weak signal is received by the trace 22 and appendages 22a. This signal or current in trace 21 and appendages 21a generates a corresponding electric field in the vicinity thereof. Still, because of the dielectric barrier between trace 21 and appendages 21a and trace 22 and appendages 22a, no signal or a very weak signal is received by trace 22 and appendages 22a.

If any liquid present in the food preservation container 75 during the evacuation operation is drawn towards the vacuum trough 10, it must pass over the traces 21, 22 and appendages 21a and 22a. The presence of the liquid in the spaces between the traces 21, 22 and appendages 21a and 22a changes the permittivity of air in the spaces. This reduced permittivity allows the electric field created by the weak signal provided to trace 21 and appendages 21a to induce a current or increases signal strength in trace 22 and appendages 22a. This induced current or received signal is provided to the microprocessor M which compares the signal to a threshold signal level. If the received signal is equal to or greater than the threshold signal level, this means that there is liquid in the food preservation container 75 about to be drawn into the vacuum trough 10. The microprocessor M may use this signal to modify control programs for the vacuum motor 17 and the heat sealing element 15. For example, upon detecting the presence of liquid in the food preservation container 75 the microprocessor M may automatically extend the amount of time the heat sealing element 15 is energized to improve seal quality. This minimizes or eliminates the need for a liquid drip tray in the vacuum trough 10. Alternately, the microprocessor M may shut off or slow the vacuum motor 17 so liquid is drawn more slowly or prevented from being drawn from the food preservation container 75 into the vacuum trough. The foregoing examples are not meant to be limiting as other possible modifications to the evacuating and sealing operations are possible.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims. A similar or nearly identical food preservation appliance is disclosed in U.S. patent application serial no. 14/126,692 filed December 16, 2013, owned by a common assignee.

## Claims

1. A food storage appliance (100), comprising:
a housing (4);
an elongated vacuum trough (10) disposed in the housing (4) configured to receive an unsealed end of a food storage container (75);
one or more electronic components including a vacuum motor (17) fluidly connected to the vacuum trough (10) and a heat sealing element (15) disposed in proximity of the vacuum trough (10);
**characterised in** further comprising:
a sensor (20) disposed on the housing (4) in proximity to the vacuum trough (10) configured to generate an electrical signal if liquid is detected in the food storage container (75) before the liquid is drawn into the vacuum trough (10) during an evacuation operation; and
an integrated electronic circuit connected to the sensor (20) configured to receive the electrical signal and configured to control one of the vacuum motor (17) or the heat sealing element (15) when liquid is detected in the food storage container (75).

2. The food storage appliance (100) of claim 1, further including the integrated electronic circuit being a microprocessor.

3. The food storage appliance (100) of claim 1, the sensor (20) further including:
an elongated non-conducting substrate having an elongated first electrical trace having a first plurality of appendages formed thereon operating as a transmitting antenna spaced apart from an elongated second electrical trace formed thereon with a second plurality of appendages operating as a receiving antenna interleaved with and spaced apart from the first plurality of appendages,
wherein the first electrical trace and the first plurality of appendages transmits an electrical signal that is received by the second electrical trace and the second plurality of appendages if liquid in the food storage container is brought into the space between the first plurality of appendages and the second plurality of appendages during the evacuation operation.

4. The food storage appliance (100) of claim 3, wherein a time the heat sealing element (15) is energized for heat sealing is controlled by the integrated electronic circuit and dependent upon whether the second electrical trace and the second plurality of appendages receive the electrical signal above a threshold signal strength from the first electrical trace and the first plurality of appendages.

5. The food storage appliance (100) of claim 3, the first trace, the first plurality of appendages, the second trace and the second plurality of appendages being comprised of metal.

6. The food storage appliance (100) of claim 5, wherein the metal is selected from the group consisting of copper, silver, aluminum and gold.

7. The food storage appliance (100) of claim 1, wherein the integrated circuit is configured to compare the first signal with the second signal and based on a determined differential to control processing of the food storage container (75) by the food preservation appliance (100).

8. A method of storing and preserving food in a food storage container (75) using a food storage appliance (100), comprising:
inserting a portion of the food storage container (75) into a vacuum trough (10) of the food storage appliance (100);
commence evacuating the food storage container (75) using a vacuum motor (17) disposed in the food storage appliance (100) fluidly connected to the vacuum trough (10);
using a sensor (20) to generate a signal if liquid is detected in the food storage container (75) prior to the liquid being drawn into the vacuum trough (10);
using a logic circuit to compare the generated signal to a threshold signal; and
using the logic circuit to control one or both of the vacuum motor (17) evacuating the food container or a heat sealing element (15) disposed in proximity of the vacuum trough (10) to heat seal the food storage container (75) if the generated signal is above the threshold signal.

9. The method of claim 8, further including selecting the sensor (20) to include:
a substrate comprised of a non-conducting material;
a first trace comprised of a conducting material etched onto the substrate having a plurality of first appendages extending perpendicularly therefrom and spaced along a lineal length of the first trace; and
a second trace comprised of a conducting material etched onto the substrate separated from the first trace by a first space, the second trace having a plurality of second appendages extending perpendicularly therefrom and spaced along a lineal length of the second trace, the second plurality of appendages interleaved with and separated by a second space from the first plurality of appendages.

10. The method of claim 8, further including selecting the logic circuit to be an integrated circuit or a microprocessor.

11. The method of claim 9, whereby the first trace and the plurality of first appendages are a transmitting antenna.

12. The method of claim 9, whereby the second trace and the plurality of second appendages are a receiving antenna.

13. The method of claim 9, further including selecting the conducting material from the group consisting of copper, silver, aluminum and gold.

## Patentansprüche

1. Nahrungsmittelaufbewahrungsvorrichtung (100), umfassend:
ein Gehäuse (4);
einen länglichen Vakuumkanal (10), der in dem Gehäuse (4) angeordnet ist und ausgebildet ist, ein unversiegeltes Ende eines Nahrungsmittelaufbewahrungsbehälters (75) aufzunehmen;
eine oder mehrere elektronische Komponenten, einschließlich eines Vakuummotors (17), der in fluider Verbindung mit dem Vakuumkanals (10) steht, sowie eines Heißsiegelelements (15), das in der Nähe des Vakuumkanals (10) angeordnet ist;
**dadurch gekennzeichnet, dass** ferner umfasst wird:
ein Sensor (20), der auf dem Gehäuse in der Nähe des Vakuumkanals (10) angeordnet ist und ausgebildet ist, ein elektrisches Signal zu erzeugen, wenn Flüssigkeit in dem Nahrungsmittelaufbewahrungsbehälter (75) detektiert wird, bevor die Flüssigkeit während einer Vakuumierungsvorgangs in den Vakuumkanals (10) gesogen wird,; und
ein integrierter elektronischer Schaltkreis, der mit dem Sensor (20) verbunden und ausgebildet ist, das elektrische Signal zu empfangen, und ausgebildet ist, den Vakuummotor (17) und/oder das Heißsiegelelement (15) zu steuern, wenn Flüssigkeit in dem Nahrungsmittelaufnahmebehälter (75) detektiert wird.

2. Nahrungsmittelaufbewahrungsvorrichtung (100) nach Anspruch 1, ferner umfassend den integrierten elektronischen Schaltkreis, der ein Mikroprozessor ist.

3. Nahrungsmittelaufbewahrungsvorrichtung (100) nach Anspruch 1, wobei der Sensor (20) ferner umfaßt:
ein längliches nicht-leiterldes Substrat mit einer länglichen ersten Spur mit einer ersten Vielzahl von darauf ausgebildeten Anschlüssen, die als Übertragungsantenne dient und von einer länglichen zweiten Spur beabstandet ist, die mit einer zweiten Vielzahl von Anschlüssen darauf ausgebildet ist, die als Empfangsantenne dienen und von der ersten Vielzahl von Anschlüssen beabstandet ist;
wobei die erste elektrische Spur und die erste Vielzahl von Anschlüssen ein elektrisches Signal übertragen, das von der zweiten elektrischen Spur und der zweiten Vielzahl von Anschlüssen empfangen wird, wenn während des Vakuumierungsvorgangs Flüssigkeit in dem Nahrungsmittelaufbewahrungsbehälter in den Raum zwischen der ersten Vielzahl von Anschlüssen und der zweiten Vielzahl von Anschlüssen gebracht wird.

4. Nahrungsmittelaufbewahrungsvorrichtung (100) nach Anspruch 3, wobei eine Zeit, zu der das Heißsiegelelement (15) zum Heißsiegeln mit Energie versorgt wird, von dem integrierten elektronischen Schaltkreis gesteuert wird und davon abhänge, ob die zweite elektrische Spur und die zweiten Vielzahl von Anschlüssen das elektrische Signal oberhalb einer Schwellwertsignalstärke von der ersten elektrischen Spur und der ersten Vielzahl von Anschlüssen empfangen,

5. Nahrungsmittelaufbewahrungsvorrichtung (100) nach Anspruch 3, wobei die erste Spur, die erste Vielzahl von Anschlüssen, die zweite Spur und die zweite Vielzahl von Anschlüssen Metall umfassen.

6. Nahrungsmittelaufbewahrungsvorrichtung (100) nach Anspruch 5, wobei das Metall aus der Gruppe ausgewählt ist, bestehend aus Kupfer, Silber, Aluminium und/oder Gold.

7. Nahrungsmittelaufbewahrungsvorrichtung (100) nach Anspruch 1, wobei der integrierte Schaltkreis ausgebildet ist, das erste Signal mit dem zweiten Signal zu vergleichen und auf der Grundlage einer ermittelten Differenz das Verarbeiten des Nahrungsmittelaufbewahrungsbehälters (75) durch die Nahrungsmittelaufbewahrungsvorrichtung (100) zu steuern.

8. Verfahren zum Lagern und Aufbewahren von Nahrungsmitteln in einem Nahrungsmittelaufbewahrungsbehälter (75) unter Verwendung einer Nahrungsmittelaufbewahrungsvorrichtung (100), umfassend:
Einbringen eines Abschnitts des Nahrungsmittelaufbewahrungsbehälters (75) in einen Vakuumkanal (10) der Nahrungsmittelaufbewahrungsvorrichtung (100);
Beginnen des Vakuumierens des Nahrungsmittelaufbewahrungsbehälters (75) unter Verwendung eines Vakuurnrnotors (17), der in der Nahrungsmittelaufbewahrungsvorrichtung (100) angeordnet ist und in fluider Verbindung mit dem Vakuumkanal (10) steht;
Verwenden eines Sensors (20), um ein Signal zu generieren, wenn Flüssigkeit in dem Nahrungsmittelaufbewahrungsbehälter (75) detektiert wird, bevor die Flüssigkeit in den Vakuumkanal (10) gesogen wird;
Verwenden eines logischen Schaltkreises, um das generierte Signal mit einem Schwellwertsignal zu vergleichen; und
Verwenden des logischen Schaltkreises, um den Vakuummotor (17) zum Vakuumieren des Nahrungsmittelbehälters oder ein Heißsiegelelement (15) zu steuern, das in der Nähe des Vakuumkanal (10) angeordnet ist, um den Nahrungsmittelaufbewahrungsbehälter (75) zu versiegeln, falls das generierte Signal größer als das Schwellwertsignal ist.

9. Verfahren nach Anspruch 8, wobei das Verfahren das Auswählen des Sensors (20) umfasst, der umfasst:
ein Substrat, das ein nicht-leitendes Material umfaßt;
eine erste Spur, die ein leitendes Material umfasst, das auf das Substrat geätzt ist und eine Vielzahl von ersten Anschlüssen umfaßt, die sich senkrecht davon erstrecken und entlang einer geraden Länge der ersten Spur beabstandet sind; und
eine zweite Spur, die ein leitfähiges Material umfasst, das auf das Substrat geätzt ist und von der ersten Spur durch einen ersten Abstand beabstandet ist, wobei die zweite Spur eine Vielzahl von zweiten Anschlüssen aufweist, die sich davon senkrecht erstrecken und entlang einer geraden Länge der zweiten Spur beabstandet sind, wobei die zweite Vielzahl von Anschlüssen mit der ersten Vielzahl von Anschlüssen vermascht ist und durch einen zweiten Abstand von der ersten Vielzahl von Anschlüssen beabstandet ist.

10. Verfahren nach Anspruch 8, ferner umfassend das Auswählen des logischen Schaltkreises, der ein integrierter Schaltkreis oder ein Mikroprozessor ist.

11. Verfahren nach Anspruch 9, wobei die erste Spur und die Vielzahl von ersten Anschlüssen eine Übertragungsantenne sind.

12. Verfahren nach Anspruch 9, wobei die zweite Spur und die Vielzahl von zweiten Anschlüssen eine Empfangsantenne sind.

13. Verfahren nach Anspruch 9, ferner umfassend das Auswählen des leitfähigen Materials aus der Gruppe bestehend aus Kupfer, Silber, Aluminium und/oder Gold.

## Revendications

1. Appareil de stockage d'aliments (100), comprenant:
un boîtier (4);
une gouttière pour vide allongée (10) disposée dans le boîtier (4) configuré pour recevoir une extrémité non scellée d'un emballage de stockage d'aliments (75);
un ou plusieurs composants électroniques comprenant un moteur pour vide (17) avec liaison fluidique à la gouttière pour vide (10) et un élément de scellement à chaud (15) disposé à proximité de la gouttière pour vide (10);
**caractérisé en ce qu'**il comprend en outre:
un détecteur (20) disposé sur le boîtier (4) à proximité de la gouttière pour vide (10), configuré pour délivrer un signal électrique si un liquide est détecté dans l'emballage de stockage d'aliments (75) avant que le liquide soit aspiré dans la gouttière pour vide (10) lors d'une opération de mise sous vide; et
un circuit électronique intégré relié au détecteur (20) configuré pour recevoir le signal électrique et configuré pour commander l'un parmi le moteur pour vide (17) ou l'élément de scellage à chaud (15) lorsque du liquide est détecté dans l'emballage de stockage d'aliments (75).

2. Appareil de stockage d'aliments (100) selon la revendication 1, comprenant en outre le circuit électronique intégré qui est un microprocesseur.

3. Appareil de stockage d'aliments (100) selon la revendication 1, le détecteur (20) comprenant en outre:
un substrat non-conducteur allongé ayant une première piste électrique allongée possédant une première pluralité d'appendices formés sur elle, agissant comme antenne de transmission, située à distance d'une deuxième piste électrique allongée formée sur celle-ci, ayant une deuxième pluralité d'appendices opérant comme antenne de réception, intercalée avec et séparée de la première pluralité d'appendices,
tandis que la première piste électrique et la première pluralité d'appendices transmettent un signal électrique qui est reçu par la deuxième piste électrique et la deuxième pluralité d'appendices, si du liquide dans l'emballage de stockage d'aliments est introduit dans l'espace entre la première pluralité d'appendices et la deuxième pluralité d'appendices lors de l'opération de mise sous vide.

4. Appareil de stockage d'aliments (100) selon la revendication 3, dans lequel le temps pendant lequel l'élément de scellage à chaud (15) est alimenté en courant pour scellage à chaud est commandé par le circuit électronique intégré et dépend de ce que la deuxième piste électrique et la deuxième pluralité d'appendices reçoivent ou non le signal électrique supérieur à une intensité de signal seuil provenant de la première piste électrique et de la première pluralité d'appendices.

5. Appareil de stockage d'aliments (100) selon la revendication 3, dans lequel la première piste, la première pluralité d'appendices, la deuxième piste et la deuxième pluralité d'appendices sont composés de métal.

6. Appareil de stockage d'aliments (100) selon la revendication 5, dans lequel le métal est choisi dans le groupe consistant en cuivre, argent, aluminium et or.

7. Appareil de stockage d'aliments (100) selon la revendication 1, dans lequel le circuit intégré est configuré pour comparer le premier signal avec le deuxième signal et, sur la base d'un différentiel déterminé, de commander le traitement de l'emballage de stockage d'aliments (75) par l'appareil de conservation d'aliments (100).

8. Procédé pour le stockage et la conservation d'aliments dans un emballage de conservation d'aliments (75) utilisant un appareil de stockage d'aliments (100), comprenant:
l'insertion d'une portion de l'emballage de stockage d'aliments (75) dans une gouttière pour vide (10) de l'appareil de stockage d'aliments (100);
le commencement de la mise sous vide de l'emballage de stockage d'aliments (75) au moyen d'un moteur pour vide (17) disposé dans l'appareil de stockage d'aliments (100), en relation fluidique avec la rigole pour vide (10);
l'utilisation d'un détecteur (20) pour générer un signal si du liquide est détecté dans l'emballage de stockage d'aliments (75) avant que le liquide soit entraîné dans la rigole pour vide (10);
l'utilisation d'un circuit logique pour comparer le signal généré avec un signal de seuil; et
l'utilisation du circuit logique pour commander l'un ou les deux des moteurs pour vide (17) mettant l'emballage d'aliments sous vide ou un élément de scellage à chaud (15) disposé à proximité de la rigole pour vide (10) pour sceller à chaud l'emballage de stockage d'aliments (75) si le signal généré est supérieur au signal de seuil.

9. Procédé selon la revendication 8, comprenant en outre la sélection du détecteur (20) pour qu'il comprenne:
un substrat composé d'une matière non-conductrice;
une première piste composée d'une matière conductrice soudée sur le substrat, ayant une pluralité de premiers appendices s'étendant perpendiculairement par rapport à elle et espacés le long d'une longueur linéaire de la première piste; et
une deuxième piste composée d'une matière conductrice soudée sur le substrat séparé de la première piste par un premier espace, la deuxième piste ayant une pluralité de deuxièmes appendices s'étendant perpendiculairement par rapport à elle et espacés le long de la longueur linéaire de la deuxième piste, la deuxième pluralité d'appendices étant intercalée avec et séparée par un deuxième espace de la première pluralité d'appendices.

10. Procédé selon la revendication 8, comprenant en outre la sélection du circuit logique comme étant un circuit intégré ou un microprocesseur.

11. Procédé selon la revendication 9, par lequel la première piste et la pluralité de premiers appendices sont une antenne de transmission.

12. Procédé selon la revendication 9, par lequel la deuxième piste et la pluralité de deuxièmes appendices sont une antenne de déception.

13. Procédé selon la revendication 9, comprenant en outre le choix du matériau conducteur dans le groupe consistant en cuivre, argent, aluminium et or.
